# EUROPEAN PATENT SPECIFICATION

(11) **EP 2 934 264 B1**
(45) Date of publication and mention of the grant of the patent: **30.05.2018**
(21) Application number: 13866125.1
(22) Date of filing: 19.11.2013
(51) Int. Cl.: A47K 10/42, B65D 83/08, A61K 8/368, D04H 1/64, A61K 8/365

(54) **WET WIPES WITH IMPROVED STRENGTH AND DISPERSIBILITY**
FEUCHTTÜCHER MIT ERHÖHTER FESTIGKEIT UND DISPERGIERBARKEIT
LINGETTES HUMIDES PRÉSENTANT UNE RÉSISTANCE ET UNE DISPERSIBILITÉ AMÉLIORÉES

(30) Priority: 21.12.2012 US 201213723614
(43) Date of publication of application: 28.10.2015
(73) Proprietor: Kimberly-Clark Worldwide, Inc., Neenah, Wisconsin 54956 (US)
(72) Inventor: HURLEY, Steven Michael, Neenah, Wisconsin 54956 (US); BUNYARD, William Clayton, DePere, Wisconsin 54115 (US); CHO, YoonHee, Yongin-si 466-583 (KR); LEE, WanDuk, Appleton, Wisconsin 54913 (US)
(74) Representative: Beacham, Annabel Rose
(86) International application number: PCT/IB2013/060255
(87) International publication number: WO 2014/097012

(56) References cited:
- EP-A1- 1 285 985
- JP-A- H09 132 896
- US-A1- 2002 155 281
- US-A1- 2011 290 437
- US-A1- 2011 293 931
- US-B2- 7 157 389

## Description

### FIELD OF THE DISCLOSURE

The present disclosure is directed to wetting compositions having a low concentration of inorganic salts, such as sodium chloride, that may be suitable for use with ion-sensitive or triggerable, water-dispersible or water-soluble cationic polymers and polymer formulations in disposable personal care articles. The present disclosure is also directed to a method of making the wetting compositions and their applicability for use with disposable personal care articles. The present disclosure is further directed to disposable personal care articles, such as wet-wipes, comprising the wetting composition in combination with ion-sensitive or triggerable, water-dispersible binder compositions.

### BACKGROUND OF THE DISCLOSURE

For many years, the problem of disposability has plagued industries that provide disposable items, such as wet wipes. In the infant care area, such wet wipes are made of nonwoven synthetic fibers, such as polypropylene, which are unaffected by the water content in the wipe. Hence, these products can be stored and used without concern about the wet strength of the wipe. Unfortunately, this desirable characteristic can also be a detriment, since these wipes cannot be safely disposed by flushing them down the toilet.

Ideally, when a disposable product is intended to be discarded in either sewer or septic systems, the product should "disperse" and thus sufficiently dissolve or disintegrate in water so as to not present problems under conditions typically found in either household or municipal systems. While much headway has been made in addressing this problem, one of the weak links has been the inability to create an economical coherent fibrous web, which will readily dissolve or disintegrate in water, but still have sufficient in-use properties such as strength, thickness, opacity, absorbency, softness, flexibility, cleansing, ease-of-use, etc. to make consumer acceptable products. Without such a product, the ability of the user to dispose of the product by flushing it down the toilet is greatly reduced, if not eliminated.

Recent binder compositions for use in these products have been developed which can be more dispersible and are more environmentally responsible than past binder compositions. Ion-sensitive binders of interest include a class of binders, which include ion-sensitive or triggerable, water-dispersible or water soluble cationic polymers and polymer formulations, such as those disclosed in U.S. Pat. No. 7,157,389 assigned to Kimberly Clark.

Disposable personal care articles, such as wet wipes also commonly include a wetting solution (also referred to herein as a wetting composition) such as is described in U.S. Pat. No. 7,157,389, (assigned to Kimberly-Clark), which may provide the desired moisture to the personal care article as well as act with the binder composition to provide the desired strength and dispersibility. Such wetting compositions, however, typically include certain amounts of salt, and in particular inorganic salts such as sodium chloride, which may limit the ability to include certain benefit ingredients in the personal care products for providing smoothness, softness and other tactile properties as well as other skin health benefits as these ingredients may be difficult to include in formulations and emulsions when sodium chloride is present (i.e., certain benefit ingredients are relatively salt-intolerant) .

As such, there exists a need for dispersible products possessing softness, flexibility, three dimensionality, and resiliency; wicking and structural integrity in the presence of body fluids (including feces) at body temperature; and true fiber dispersion after toilet flushing so that product does not become entangled with tree roots or at bends in sewer pipes. Moreover, there is a need for water-dispersible, flushable wet wipes that are stable during storage, retain a desired level of wet strength during use, and are wetted with a wetting composition that is relatively free, or is substantially free, of salts such as sodium chloride. Such a product is needed at a reasonable cost without compromising product safety and environmental concerns, something that past products have failed to do.

### BRIEF DESCRIPTION OF THE DRAWINGS

Figure 1 is graph depicting the solubility of a binder composition in a sodium chloride solution.
Figure 2 is a graph depicting the dispersibility of a binder composition in a sodium citrate solution as described in Example 1.

### SUMMARY OF THE DISCLOSURE

The present disclosure is directed to wetting compositions, which have been developed to address the above-described problems and to be compatible for use with ion-sensitive or triggerable binder compositions. The combination of the wetting compositions and the binder compositions provides an optimum level of wet strength through the use of at least one polyprotic acid having three or more functional groups and benzoic acid as the sole or primary triggering agents in the wetting composition, while not requiring the use of sodium chloride or other inorganic salts. When the wet wipe is discarded into a wastewater stream, the concentrations of the polyprotic acid and benzoic acid are diluted, the binder becomes soluble, and the strength drops below a critical level such to disperse the wipe.

The wetting compositions of the present disclosure are useful for air-laid and wet-laid nonwoven fabrics for applications, such as body-side liners, fluid distribution materials, fluid in-take materials (surge) or cover stock in various personal care products. The polymer formulations of the present disclosure are particularly useful as a wetting composition for flushable personal care products, particularly wet wipes for personal use, such as cleaning or treating skin, make-up removal, nail polish removal, medical care, and also wipes for use in hard surface cleaning, automotive care, including wipes comprising cleaning agents, disinfectants, and the like. The flushable products maintain integrity or wet strength during storage and use, and break apart or disperse after disposal in the toilet. Suitable substrates, or articles, for treatment include tissue, such as creped or uncreped tissue, coform products, hydroentangled webs, airlaid mats, fluff pulp, nonwoven webs, and composites thereof.

Specifically, in one aspect, a wet wipe comprising a fibrous material, an ion-triggerable cationic binder composition, and a wetting composition is disclosed. The fibrous material has a strength of at least 300 g/in and a dispersibility of no more than 100 g/in. The wetting composition comprises at least one polyprotic acid having three or more functional groups and benzoic acid, and the ratio of the benzoic acid to the at least one polyprotic acid is greater than 2.5.

In another aspect, a wet wipe comprising a fibrous material, an ion-triggerable cationic binder composition, and a wetting composition is disclosed. The fibrous material has a strength of at least 300 g/in and a dispersibility of no more than 100 g/in. The wetting composition comprises at least one polyprotic acid having three or more functional groups and benzoic acid, and the pH of the wetting composition after expression from the wet wipe is from about 4.5 to about 6.0.

In a further aspect, a wet wipe comprising a fibrous material, an ion-triggerable cationic binder composition, and a wetting composition is disclosed. The fibrous material has a strength of at least 300 g/in and a dispersibility of no more than 100 g/in. The wetting composition comprises at least one polyprotic acid having three or more functional groups, benzoic acid, behentrimonium methosulfate, cetearyl alcohol, and butylene glycol. The ratio of the benzoic acid to the at least one polyprotic acid is greater than 2.5.

These and other objects, features and advantages of the present disclosure will become apparent after a review of the following detailed description of the disclosed embodiments and the appended claims.

### DETAILED DESCRIPTION OF THE DISCLOSED EMBODIMENTS

The present disclosure is practiced using wetting compositions that include at least one polyprotic acid having three or more functional groups and also include benzoic acid. The wetting compositions are used in combination with binder compositions that include triggerable cationic polymers or polymer compositions that are ion-sensitive. In order to be an effective ion-sensitive or triggerable cationic polymer or cationic polymer binder formulation suitable for use in flushable or water-dispersible personal care products, the binder formulations should desirably be (1) functional; i.e., maintain wet strength under controlled conditions and dissolve or disperse in a reasonable period of time in soft or hard water, such as found in toilets and sinks around the world; (2) safe (not toxic); and (3) relatively economical. In addition to the foregoing factors, the ion-sensitive or triggerable binder formulations when used as a binder composition with the wetting composition on a non-woven substrate, such as a wet wipe, desirably should be (4) processable on a commercial basis; i.e., may be applied relatively quickly on a large scale basis, such as by spraying (which thereby requires that the binder composition have a relatively low viscosity at high shear); (5) provide acceptable levels of sheet or substrate wettability; (6) provide reduced levels of sheet stiffness; and (7) reduced tackiness. The wetting composition with which the wet wipes of the present disclosure are treated can provide some of the foregoing advantages, and, in addition, can provide one or more of (8) improved skin care, (9) improved tactile properties, and (10) promote good cleaning by providing a balance in use between friction and lubricity on the skin (skin glide). In addition, because the wetting compositions comprise a reduced amount of sodium chloride due to the presence of the polyprotic acid and the benzoic acid, the ability to include certain optional benefit ingredients may be improved. The wetting compositions and binder formulations of the present disclosure and articles made therewith, especially wet wipes comprising particular wetting compositions set forth below, can meet many or all of the above criteria. Of course, it is not necessary for all of the advantages of the preferred embodiments of the present disclosure to be met to fall within the scope of the present disclosure.

### Wet Wipe Wetting Composition and Wet Wipes Containing the Same

One particularly interesting embodiment of the present disclosure is the production of pre-moistened wipes, or wet wipes, including flushable or disposable wipes, from the below-described triggerable binder compositions and fibrous materials. For wipes, the fibrous material may be in the form of a woven or nonwoven fabric; however, nonwoven fabrics are more suitable. The nonwoven fabric is suitably formed from relatively short fibers, such as wood pulp fibers. The minimum length of the fibers depends on the method selected for forming the nonwoven fabric. Where the nonwoven fabric is formed by a wet or dry method, the fiber length is suitably from about 0.1 millimeters to 15 millimeters. Suitably, the nonwoven fabric has a relatively low wet cohesive strength when it is not bonded together by an adhesive or binder material. When such nonwoven fabrics are bonded together by a binder composition, which loses its bonding strength in tap water and in sewer water, the fabric will break up readily by the agitation provided by flushing and moving through the sewer pipes.

The finished wipes may be individually packaged, suitably in a folded condition, in a moisture proof envelope or packaged in containers holding any desired number of sheets in a water-tight package with a wetting composition applied to the wipe. The finished wipes may also be packaged as a roll of separable sheets in a moisture-proof container holding any desired number of sheets on the roll with a wetting composition applied to the wipes. The roll can be coreless and either hollow or solid. Coreless rolls, including rolls with a hollow center or without a solid center, can be produced with known coreless roll winders, including those of SRP Industry, Inc. (San Jose, Calif.); Shimizu Manufacturing (Japan), and the devices disclosed in U.S. Pat. No. 4,667,890, issued May 26, 1987 to Gietman. Solid-wound coreless rolls can offer more product for a given volume and can be adapted for a wide variety of dispensers.

Relative to the weight of the dry fabric, the wipe may suitably contain from about 10 percent to about 400 percent of the wetting composition, more suitably from about 100 percent to about 300 percent of the wetting composition, and even more suitably from about 180 percent to about 240 percent of the wetting composition. The wipe maintains its desired characteristics over the time periods involved in warehousing, transportation, retail display and storage by the consumer. Accordingly, shelf life may range from two months to two years.

Various forms of impermeable envelopes and storage means for containing wet-packaged materials, such as wipes and towelettes and the like, are well known in the art. Any of these may be employed in packaging the pre-moistened wipes of the present disclosure.

Suitably, the pre-moistened wipes of the present disclosure are wetted with an aqueous wetting composition, which has one or more of the following properties:
(1) is compatible with the below-described triggerable binder compositions;
(2) enables the pre-moistened wipe to maintain its wet strength during converting, storage and usage (including dispensing), as well as, dispersibility in a toilet bowl;
(3) reduces tackiness of the wipe, and provides tactile properties, such as skin glide and a "lotion-like feel"; and
(4) acts as a vehicle to deliver "moist cleansing" and other skin health benefits.

One aspect of the present disclosure is a wetting composition, which contains an insolubilizing agent that maintains the strength of a water-dispersible binder until the insolubilizing agent is diluted with water, whereupon the strength of the water-dispersible binder begins to decay. The water-dispersible binder may be any of the triggerable binder compositions described below or any other cationic triggerable binder composition known in the art. The insolubilizing agent in the wetting composition includes at least one polyprotic acid having three or more functional groups in combination with benzoic acid, the combination of which provides in-use and storage strength to the water-dispersible binder composition, and can be diluted in water to permit dispersion of the substrate as the binder polymer triggers to a weaker state. In one embodiment, the polyprotic acid having at least three functional groups is citric acid and salts thereof (including, but not limited to, sodium citrate, disodium citrate, monosodium citrate, potassium citrate, dipotassium citrate, monopotassium citrate, lithium citrate, dilithium citrate, monolithium citrate, magnesium citrate, calcium citrate), EDTA, or combinations thereof. Further, benzoic acid, as used herein, refers to benzoic acid as well as salts thereof, including, but not limited to, sodium benzoate, potassium benzoate, lithium benzoate, calcium benzoate, and magnesium benzoate.

Suitably, the wetting composition contains from about 0.10 wt% to about 0.35 wt% of the polyprotic acid, including from about 0.20 wt% to about 0.35 wt%, and including about 0.20 wt%. The wetting composition further contains from about 0.60 wt% to about 1.30 wt% of the benzoic acid, including from about 0.8 wt% to about 1.30 wt%, including 0.8 wt% to about 1.2 wt%, including from about 0.8 wt% to about 1.1 wt%, and including from about 0.8 wt% to about 1.0 wt%. In one particular embodiment, the wetting composition includes about 0.2 wt% sodium citrate, about 0.05 wt% citric acid, about 0.8 wt% sodium benzoate, and about 0.2 wt% benzoic acid. In addition, the weight ratio of the benzoic acid to the polyprotic acid in the wetting composition is greater than 2.5, including greater than 3.0, including from about 3.0 to about 10, including from about 3.3 to about 7.5, including from about 4 to about 5, and including from about 4 to about 4.5.

In one embodiment, the wetting composition is substantially free of sodium chloride. Specifically, the wetting composition may include less than 2 wt%, less than 1.5 wt%, less than 1.0 wt%, less than 0.75 wt%, less than 0.5 wt%, less than 0.25 wt%, less than 0.1 wt%, less than 0.05 wt% sodium chloride, or even 0 wt% sodium chloride. In one embodiment, the wetting composition includes less than 0.5 wt%, including less than 0.3 wt%.

Suitably, the pH range of the wetting composition is from about 3.5 to about 7.5. More suitably, the pH range of the wetting composition is from about 4 to about 6. Suitably, the overall pH of the wet wipe product; i.e., the complete wet wipe product including the fabric portion and the wetting solution portion, is from about 4.5 to about 5.5; suitably, about 5.0. In addition, the pH of the liquid included on a wet wipe after expression from a formed wet wipe (i.e., "expressed pH") may be from about 4.5 to about 7, including from about 4.5 to about 6, including from about 5 to about 6, including from about 5 to about 5.5, and including about 5.2. The wetting composition further has an ionic strength of greater than 0.6 mol/dm³, including, greater than 0.7 mol/dm³, including greater than 0.75 mol/dm³.

It has been found that by combining the benzoic acid and polyprotic acid in wetting compositions having an expressed pH as described above, improved skin health can be provided, as well as providing a balanced number of anions and acid to provide improved wet strength and stability to personal care articles (e.g., wet wipes) including the wetting compositions.

Another advantage of the above-described combination of components for use in the wetting compositions is that these components allow for the use of emulsions and other formulations including salt-intolerant components, which provide further enhanced skin feel. That is, in some embodiments, emulsions and other salt-intolerant component-containing formulations that are generally not available with 2% sodium chloride can be prepared with the wetting compositions of the present disclosure. Exemplary such components include dicaprylyl ether (and) decyl Glucoside (and) glyceryl Oleate (available as Plantasil® Micro from Cognis GmbH, Germany) and behentrimonium methosulfate (and) cetearyl alcohol (and) butylene glycol (available as Incroquat™ Behenyl TMS-50 from Croda, United Kingdom).

The wetting composition of the present disclosure may further comprise a variety of additives compatible with the insolubilizing agent and the water-dispersible binder, such that the strength and dispersibility functions of the wipe are not jeopardized. By reducing or eliminating the amount of sodium chloride in the wetting composition through the use of the combination of polyprotic acid and benzoic acid as the triggering agent, actives, or benefit ingredients, that otherwise would not have been compatible in the wetting composition may be utilized. Suitable additives in the wetting composition include, but are not limited to, the following additives: skin-care additives; odor control agents; detackifying agents to reduce the tackiness of the binder; particulates; antimicrobial agents; preservatives; wetting agents and cleaning agents, such as detergents, surfactants, some silicones; emollients; surface feel modifiers for improved tactile sensation (e.g., lubricity) on the skin; fragrance; fragrance solubilizers; opacifiers; fluorescent whitening agents; UV absorbers; pharmaceuticals; and pH control agents, such as malic acid or potassium hydroxide.

### Skin-Care Additives

As used herein, the term "skin-care additives" represents additives, which provide one or more benefits to the user, such as a reduction in the probability of having diaper rash and/or other skin damage caused by fecal enzymes. These enzymes, particularly trypsin, chymotrypsin and elastase, are proteolytic enzymes produced in the gastrointestinal tract to digest food. In infants, for example, the feces tend to be watery and contain, among other materials, bacteria, and some amounts of undegraded digestive enzymes. These enzymes, if they remain in contact with the skin for any appreciable period of time, have been found to cause an irritation that is uncomfortable in itself and can predispose the skin to infection by microorganisms. As a countermeasure, skin-care additives include, but are not limited to, the enzyme inhibitors and sequestrants set forth hereafter. The wetting composition may contain less than 5 weight percent of skin-care additives based on the total weight of the wetting composition. More specifically, the wetting composition may contain from about 0.01 weight percent to about 2 weight percent of skin-care additives. Even more specifically, the wetting composition may contain from about 0.01 weight percent to about 0.05 weight percent of skin-care additives.

A variety of skin-care additives may be added to the wetting composition and the pre-moistened wipes of the present disclosure or included therein. In one embodiment of the present disclosure, skin-care additives in the form of particles are added to serve as fecal enzyme inhibitors, offering potential benefits in the reduction of diaper rash and skin damage caused by fecal enzymes. U.S. Pat. No. 6,051,749, issued Apr. 18, 2000 to Schulz et al., discloses organophilic clays in a woven or nonwoven web, said to be useful for inhibiting fecal enzymes. Such materials may be used in the present disclosure, including reaction products of a long chain organic quaternary ammonium compound with one or more of the following clays: montmorillonite, bentonite, beidellite, hectorite, saponite, and stevensite.

Many other skin-care additives may be incorporated into the wetting composition and pre-moistened wipes of the present disclosure, including, but not limited to, sun blocking agents and UV absorbers, acne treatments, pharmaceuticals, baking soda (including encapsulated forms thereof), vitamins and their derivatives such as Vitamins A or E, botanicals such as witch hazel extract and aloe vera, allantoin, emollients, disinfectants, hydroxy acids for wrinkle control or anti-aging effects, sunscreens, tanning promoters, skin lighteners, deodorants and antiperspirants, ceramides for skin benefits and other uses, astringents, moisturizers, nail polish removers, insect repellants, antioxidants, antiseptics, anti-inflammatory agents and the like, provided that the additives are compatible with an ion-sensitive binder composition associated therewith, and especially the ion-sensitive binder compositions (i.e., they do not cause a substantial loss of strength in the wet state of the pre-moistened wipes, prior to dilution in water, while permitting dispersibility in water).

Useful materials for skin care and other benefits are listed in McCutcheon's 1999, Vol. 2: Functional Materials, MC Publishing Company, Glen Rock, N.J. Many useful botanicals for skin care are provided by Active Organics, Lewisville, Tex.

### Preservatives and Anti-Microbial Agents

The wetting composition of the present disclosure may also contain preservatives and/or anti-microbial agents. Several preservatives and/or anti-microbial agents, such as Neolone 950 (Methylisothiazolinone, available from Rohm and Haas, Philadelphia, Pa.) and Mackstat H 66 (available from McIntyre Group, Chicago, I11.), have been found to give excellent results in preventing bacteria and mold growth. Other suitable preservatives and anti-microbial agents include, but are not limited to DMDM hydantoin (e.g., Glydant Plus™, Lonza, Inc., Fair Lawn, N.J.), iodopropynyl butylcarbamate, Kathon (Rohm and Hass, Philadelphia, Pa.), methylparaben, propylparaben, 2-bromo-2-nitropropane-1,3-diol, benzoic acid, benzalkonium chloride, benzethonium chloride, and the like. Sodium benzoate may also be included as a preservative. Suitably, the wetting composition contains less than 2 weight percent on an active basis of preservatives and/or anti-microbial agents based on the total weight of the wetting composition. More suitably, the wetting composition contains from about 0.01 weight percent to about 1 weight percent of preservatives and/or anti-microbial agents. Even more suitably, the wetting composition contains from about 0.01 weight percent to about 0.5 weight percent of preservatives and/or anti-microbial agents.

### Wetting Agents and Cleaning Agents

A variety of wetting agents and/or cleaning agents may be used in the wetting composition of the present disclosure. Suitable wetting agents and/or cleaning agents include, but are not limited to, detergents and nonionic, amphoteric, zwitterionic, and cationic surfactants. It should be understood by one skilled in the art that the surfactant should be chosen such to not negatively impact the wet strength and/or dispersibility of the end products. Suitably, the wetting composition contains less than about 3 weight percent of wetting agents and/or cleaning agents based on the total weight of the wetting composition. More suitably, the wetting composition contains from about 0.01 weight percent to about 2 weight percent of wetting agents and/or cleaning agents. Even more suitably, the wetting composition contains from about 0.1 weight percent to about 0.5 weight percent of wetting agents and/or cleaning agents. Suitable cationic surfactants may include, but are not limited to, quaternary ammonium alkyl halides like cetyl trimethyl ammonium chloride and cetyl trimethyl ammonium bromide.

Amino acid-based surfactant systems, such as those derived from amino acids L-glutamic acid and other natural fatty acids, offer pH compatibility to human skin and good cleansing power, while being relatively safe and providing improved tactile and moisturization properties compared to other anionic surfactants. One function of the surfactant is to improve wetting of the dry substrate with the wetting composition. Another function of the surfactant can be to disperse bathroom soils when the pre-moistened wipe contacts a soiled area and to enhance their absorption into the substrate. The surfactant can further assist in make-up removal, general personal cleansing, hard surface cleansing, odor control, and the like. One commercial example of an amino-acid based surfactant is acylglutamate, marketed under the Amisoft name by Ajinomoto Corp., Tokyo, Japan.

Suitable non-ionic surfactants include, but are not limited to, the condensation products of ethylene oxide with a hydrophobic (oleophilic) polyoxyalkylene base formed by the condensation of propylene oxide with propylene glycol. The hydrophobic portion of these compounds desirably has a molecular weight sufficiently high so as to render it water-insoluble. The addition of polyoxyethylene moieties to this hydrophobic portion increases the water-solubility of the molecule as a whole, and the liquid character of the product is retained up to the point where the polyoxyethylene content is about 50% of the total weight of the condensation product. Examples of compounds of this type include commercially-available Pluronic surfactants (BASF Wyandotte Corp.), especially those in which the polyoxypropylene ether has a molecular weight of about 1500-3000 and the polyoxyethylene content is about 35-55% of the molecule by weight, i.e. Pluronic L-62.

Other useful nonionic surfactants include, but are not limited to, the condensation products of C₈-C₂₂ alkyl alcohols with 2-50 moles of ethylene oxide per mole of alcohol. Examples of compounds of this type include the condensation products of C₁₁-C₁₅ secondary alkyl alcohols with 3-50 moles of ethylene oxide per mole of alcohol, which are commercially-available as the Poly-Tergent SLF series from Olin Chemicals or the TERGITOL® series from Union Carbide; i.e., TERGITOL® 25-L-7, which is formed by condensing about 7 moles of ethylene oxide with a C₁₂-C₁₅ alkanol.

Other nonionic surfactants, which may be employed in the wetting composition of the present disclosure, include the ethylene oxide esters of C₆-C₁₂ alkyl phenols such as (nonylphenoxy)polyoxyethylene ether. Particularly useful are the esters prepared by condensing about 8-12 moles of ethylene oxide with nonylphenol, i.e. the IGEPAL® CO series (GAF Corp.).

Further non-ionic surfactants (also referred to as surface active agents) include, but are not limited to, alkyl polyglycosides (APG), derived as a condensation product of dextrose (D-glucose) and a straight or branched chain alcohol. The glycoside portion of the surfactant provides a hydrophile having high hydroxyl density, which enhances water solubility. Additionally, the inherent stability of the acetal linkage of the glycoside provides chemical stability in alkaline systems. Furthermore, unlike some non-ionic surface active agents, alkyl polyglycosides have no cloud point, allowing one to formulate without a hydrotrope, and these are very mild, as well as readily biodegradable non-ionic surfactants. This class of surfactants is available from Horizon Chemical under the trade names of APG-300, APG-350, APG-500, and APG-500.

Additional non-ionic surfactants include, but are not limited to, lauryl glucoside (Plantacare® 1200 UP available from Cognis, Germany) and PEG-7 glyceryl cocoate (Glycerox™ HE available from Croda, Edison, New Jersey).

Suitable amphoteric surfactants include, but are not limited to, myristamidopropyl PG-dimonium chloride phosphate and water (Arlasilk™ phospholipid PTM available from Croda, Edison, New Jersey) and lineoleamidopropyl PG-dimonium chloride phosphate, propylene, and water (Arlasilk™ phospholipid EFA available from Croda, Edison, New Jersey).

Silicones are another class of wetting agents available in pure form, or as microemulsions, macroemulsions, and the like. One exemplary non-ionic surfactant group is the silicone-glycol copolymers. These surfactants are prepared by adding poly(lower)alkylenoxy chains to the free hydroxyl groups of dimethylpolysiloxanols and are available from the Dow Corning Corp as Dow Corning 190 and 193 surfactants (CTFA name: dimethicone copolyol). These surfactants function, with or without any volatile silicones used as solvents, to control foaming produced by the other surfactants, and also impart a shine to metallic, ceramic, and glass surfaces.

### Emollients

The wetting composition of the present disclosure may also contain one or more emollients. Suitable emollients include, but are not limited to, PEG 75 lanolin, methyl gluceth 20 benzoate, C₁₂-C₁₅ alkyl benzoate, ethoxylated cetyl stearyl alcohol, propylene glycol, glycerin, sorbitol, betaine products marketed as Lambent wax WS-L, Lambent WD-F, Cetiol HE (Henkel Corp.), Glucam P20 (Amerchol), Polyox WSR N-10 (Union Carbide), Polyox WSR N-3000 (Union Carbide), Luviquat (BASF), Finsolv SLB 101 (Finetex Corp.), mink oil, allantoin, stearyl alcohol, Estol 1517 (Unichema), and Finsolv SLB 201 (Finetex Corp.).

An emollient can also be applied to a surface of the article prior to or after wetting with the wetting composition. Such an emollient may be insoluble in the wetting composition and can be immobile except when exposed to a force. For example, a petrolatum-based emollient can be applied to one surface in a pattern, after which the other surface is wetted to saturate the wipe. Such a product could provide a cleaning surface and an opposing skin treatment surface.

The emollient composition in such products and other products can comprise a plastic or fluid emollient such as one or more liquid hydrocarbons (e.g., petrolatum), mineral oil and the like, vegetable and animal fats (e.g., lanolin, phospholipids and their derivatives) and/or a silicone materials such as one or more alkyl substituted polysiloxane polymers, including the polysiloxane emollients disclosed in U.S. Pat. No. 5,891,126, issued Apr. 6, 1999 to Osborn, III et al.. Optionally, a hydrophilic surfactant may be combined with a plastic emollient to improve wettability of the coated surface. In some embodiments of the present disclosure, it is contemplated that liquid hydrocarbon emollients and/or alkyl substituted polysiloxane polymers may be blended or combined with one or more fatty acid ester emollients derived from fatty acids or fatty alcohols.

In an embodiment of the present disclosure, the emollient material is in the form of an emollient blend. Suitably, the emollient blend comprises a combination of one or more liquid hydrocarbons (e.g., petrolatum), mineral oil and the like, vegetable and animal fats (e.g., lanolin, phospholipids and their derivatives), with a silicone material such as one or more alkyl substituted polysiloxane polymers. More suitably, the emollient blend comprises a combination of liquid hydrocarbons (e.g., petrolatum) with dimethicone or with dimethicone and other alkyl substituted polysiloxane polymers. In some embodiments of the present disclosure, it is contemplated that blends of liquid hydrocarbon emollients and/or alkyl substituted polysiloxane polymers may be blended with one or more fatty acid ester emollients derived from fatty acids or fatty alcohols. PEG-7 glyceryl cocoate, available as Standamul HE (Henkel Corp., Hoboken, N.J.), can also be considered.

Water-soluble, self-emulsifying emollient oils, which are useful in the present wetting compositions, include the polyoxyalkoxylated lanolins and the polyoxyalkoxylated fatty alcohols, as disclosed in U.S. Pat. No. 4,690,821, issued Sep. 1, 1987 to Smith et al.. The polyoxyalkoxy chains desirably will comprise mixed propylenoxy and ethyleneoxy units. The lanolin derivatives will typically comprise about 20-70 such lower-alkoxy units while the C₁₂-C₂₀ fatty alcohols will be derivatized with about 8-15 lower-alkyl units. One such useful lanolin derivative is Lanexol AWS (PPG-12-PEG-50, Croda, Inc., New York, N.Y.). A useful poly(15-20) C₂-C₃-alkoxylate is PPG-5-Ceteth-20, known as Procetyl AWS (Croda, Inc.).

According to one embodiment of the present disclosure, the emollient material reduces undesirable tactile attributes, if any, of the wetting composition. For example, emollient materials, including dimethicone, can reduce the level of tackiness that may be caused by the ion-sensitive binder or other components in the wetting composition, thus serving as a detackifier.

Suitably, the wetting composition contains less than about 25 weight percent of emollients based on the total weight of the wetting composition. More specifically, the wetting composition may comprise less than 5 weight percent emollient, and most specifically less than 2 weight percent emollient. More suitably, the wetting composition may contain from about 0.01 weight percent to about 8 weight percent of emollients. Even more suitably, the wetting composition may contain from about 0.2 weight percent to about 2 weight percent of emollients.

In one embodiment, the wetting composition and/or pre-moistened wipes of the present disclosure comprise an oil-in-water emulsion comprising an oil phase containing at least one emollient oil and at least one emollient wax stabilizer dispersed in an aqueous phase comprising at least one polyhydric alcohol emollient and at least one organic water-soluble detergent, as disclosed in U.S. Pat. No. 4,559,157, issued Dec. 17, 1985 to Smith et al..

### Fragrances

A variety of fragrances may be used in the wetting composition of the present disclosure. Suitably, the wetting composition contains less than 2 weight percent of fragrances based on the total weight of the wetting composition. More suitably, the wetting composition contains from about 0.01 weight percent to about 1 weight percent of fragrances. Even more suitably, the wetting composition contains from about 0.01 weight percent to about 0.05 weight percent of fragrances. Fragrance Solubilizers

Further, a variety of fragrance solubilizers may be used in the wetting composition of the present disclosure. Suitable fragrance solubilizers include, but are not limited to, polysorbate 20, propylene glycol, ethanol, isopropanol, diethylene glycol monoethyl ether, dipropylene glycol, diethyl phthalate, triethyl citrate, Ameroxol OE-2 (Amerchol Corp.), Brij 78 and Brij 98 (ICI Surfactants), Arlasolve 200 (ICI Surfactants), Calfax 16L-35 (Pilot Chemical Co.), Capmul POE-S (Abitec Corp.), Finsolv SUBSTANTIAL (Finetex), and the like. Suitably, the wetting composition contains less than 2 weight percent of fragrance solubilizers based on the total weight of the wetting composition. More suitably, the wetting composition contains from about 0.01 weight percent to about 1 weight percent of fragrance solubilizers. Even more suitably, the wetting composition contains from about 0.01 weight percent to about 0.05 weight percent of fragrance solubilizers.

Although a variety of wetting compositions, formed from one or more of the above-described components, may be used with the wet wipes of the present disclosure, in one embodiment, the wetting composition contains the following components, given in weight percent of the wetting composition, as shown in Table 1 below:

**Table 1. Wetting Composition Components**

| *Wetting Composition Component:* | *Weight Percent:* |
|---|---|
| Deionized Water | about 86 to about 98 |
| Insolubilizing agent benzoic acid and its salts and polyprotic acid and its salts in a ratio of greater than 2.5 | about 0.5 to about 2 |
| Preservative | Up to 2 |
| Surfactant | Up to 2 |
| Emollient | Up to 1 |
| Fragrance | Up to 0.3 |
| Fragrance solubilizer | Up to 0.5 |

In another embodiment of the present disclosure, the wetting composition comprises the following components, given in weight percent of the wetting composition, as shown in Table 2 below:

**Table 2. Wetting Composition Components**

| *Class of Wetting Composition Component:* | *Specific Wetting Composition Component:* | *Component Name:* | *Weight Percent:* |
|---|---|---|---|
| Vehicle | Deionized Water | | about 86 to about 98 |
| Insolubilizing agent | benzoic acid and its salts and polyprotic acid and its salts in a ratio of greater than 2.5 | | about 0.5 to about 2 |
| Preservative | Methylisothiaz olinone | Neolone 950 (Rohm and Haas, Philadelph ia, Pa) | Up to 2 |
| Surfactant | lineoleamidopr opyl PG-dimonium chloride phosphate, propylene, and water | Arlasilk™ phospholip id EFA (Croda, Edison, New Jersey) | Up to 2 |
| Emollient | Propylene glycol | | Up to 1 |
| Fragrance | Fragrance | Dragoco 0/708768 (Dragoco, Roseville, MN) | Up to 0.3 |
| Fragrance solubilizer | Polysorbate 20 | Glennsurf L20 (Glenn Corp., St. Paul, MN) | Up to 0.5 |

In another embodiment of the present disclosure, the wetting composition comprises the following components, given in weight percent of the wetting composition, as shown in Table 3 below:

**Table 3. An Exemplary Wetting Composition**

| *Class of Wetting composition Component:* | *Specific Wetting composition Component:* | *Component Name:* | *Weight Percent:* |
|---|---|---|---|
| Vehicle | Deionized Water | | about 97.3 |
| Insolubilizing agent | benzoic acid and its salts and polyprotic acids and its salts in a ration of greater than 2.5 | | about 1.2 |
| Preservative | Methylisothia zolinone | Neolone 950 (Rohm and Haas, Philadelph ia, Pa) | about 0.1 |
| Surfactant | Arlasilk™ EFA (Croda Inc., Columbus | CS22/ECS 22P | about 0.5 |
| | Circle, Edison, NJ) | | |
| Emollient | Polypropylene glycol | | about 0.5 |
| Fragrance | Fragrance | Dragoco Fragrance 0/708768 | about 0.1 |
| Fragrance solubilizer | Polysorbate 20 | Glennsurf L20 | about 0.3 |

It should be noted that the above-described wetting compositions of the present disclosure may be used with any one of the below-described triggerable binder compositions. Further, the above-described wetting compositions of the present disclosure may be used with any other binder composition, including conventional binder compositions, or with any known fibrous or absorbent substrate, whether dispersible or not.

### Strength Properties

In one embodiment of the present disclosure, wet wipes are produced using the above-described wetting composition in Table 2 and an air-laid fibrous material comprising about 75 weight percent of bleached kraft fibers and 25 weight percent of any of the above-described ion-sensitive or triggerable binder compositions, wherein the weight percentages are based on the total weight of the dry nonwoven fabric. The amount of wetting composition added to the nonwoven fabric, relative to the weight of the dry nonwoven fabric in this embodiment, is suitably about 180 percent to about 240 weight percent.

In a further embodiment of the present disclosure, wet wipes are produced using the above-described wetting composition in Table 1 and an air-laid fibrous material comprising 80 weight percent of softwood fibers and 20 weight percent of an ion-sensitive binder. The amount of wetting composition added to the nonwoven fabric, relative to the weight of the dry nonwoven fabric in this embodiment, is suitably about 180 percent to about 240 weight percent. In a further embodiment of the present disclosure, wet wipes are produced using the above-described wetting composition in Table 1 and an air-laid fibrous material comprising 90 weight percent of softwood fibers and 10 weight percent of an ion-sensitive binder. The amount of wetting composition added to the nonwoven fabric, relative to the weight of the dry nonwoven fabric in this embodiment, is suitably about 180 percent to about 240 weight percent.

Suitably, the wet wipes of the present disclosure possess an in-use wet tensile strength of at least 100 g/in, including at least 150 g/in, including 200 g/in, and including 250 g/in, when soaked with 10% to 400% by weight wet wipes solution containing more than 0.5% by weight polyprotic acids having three or more functional groups and benzoic acid such that the ratio of benzoic acid to the polyprotic acid is greater than 2.5, and a tensile strength of less than 30 g/in after being soaked in soft water or hard water containing up to 200 ppm concentration of Ca²⁺ and/or Mg²⁺ for 24 hours or less, preferably after about one hour.

More suitably, the wet wipes of the present disclosure possess an in-use machine direction ("MD") wet tensile strength of at least 300 g/in when soaked with 10% to 400% by weight wet wipes solution containing more than 0.5% by weight polyprotic acids having three or more functional groups and benzoic acid such that the ratio of benzoic acid to the polyprotic acid is greater than 2.5, and a tensile strength of less than 75 g/in after being soaked in soft water or hard water containing up to 200 ppm concentration of Ca²⁺ and/or Mg²⁺ for 24 hours or less, preferably after about one hour.

In some embodiments, such wipes can have MD wet tensile values of 250 g/in or greater, and after soaking values (typically, after about one hour soak, also referred to herein as dispersibility) of no more than 100 g/in, more specifically about 80 g/in or less, and most specifically about 50 g/in or less.

Most suitably, the wet wipes of the present disclosure possess an in-use wet tensile strength of greater than 300 g/in, such as 400 g/in, 450 g/in, 500 g/in, 600 g/in, or higher, when soaked with 10% to 400% by weight wet wipes solution containing more than 0.5% by weight polyprotic acids having three or more functional groups and benzoic acid such that the ratio of benzoic acid to the polyprotic acid is greater than 2.5, and a tensile strength of less than 30 g/in after being soaked in soft water or hard water containing up to 200 ppm concentration of Ca²⁺ and/or Mg²⁺ for 24 hours or less, preferably after about one hour.

Products with higher basis weights than flushable wet wipes may have relatively higher wet tensile strength. For example, products, such as pre-moistened towels or hard-surface cleaning wipes, may have basis weights above 70 gsm, such as from about 80 gsm to about 150 gsm. Such products can have cross-direction wet tensile ("CDWT") values of 500 g/in or greater, and after soaking values of about 150 g/in or less, more specifically about 100 g/in or less, and most specifically about 50 g/in or less.

### Ion Triggerable Cationic Polymer Compositions

The wetting composition of the wipes described herein may be used with ion triggerable cationic polymers, such as ion triggerable cationic polymers that are the polymerization product of a vinyl-functional cationic monomer, and one or more hydrophobic vinyl monomers with alkyl side chain sizes of up to 4 carbons long. In one particularly suitable embodiment, the ion triggerable cationic polymers are the polymerization product of a vinyl-functional cationic monomer, and one or more hydrophobic vinyl monomers with alkyl side chain sizes of up to four carbons long incorporated in a random manner. Additionally, a minor amount of another vinyl monomer with linear or branched alkyl groups 4 carbons or longer, alkyl hydroxy, polyoxyalkylene, or other functional groups may be employed. The ion triggerable cationic polymers function as adhesives for tissue, airlaid pulp, and other nonwoven webs and provide sufficient in-use strength (typically >300 g/in.) in solutions comprising at least one polyprotic acid having three or more functional groups and benzoic acid. The nonwoven webs are also dispersible in tap water (including hard water up to 200 ppm as metal ion), typically losing most of their wet strength (<30-75 g/in.) in 24 hours, or less.

The generic structure for the ion triggerable cationic polymers is shown below: 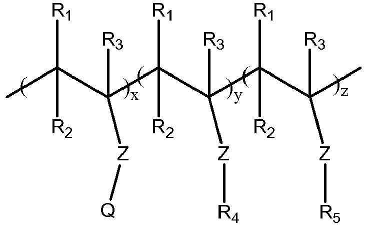 wherein x=1 to about 15 mole percent; y=about 60 to about 99 mole percent; and z=0 to about 30 mole percent; Q is selected from C₁-C₄ alkyl ammonium, quaternary C₁-C₄ alkyl ammonium and benzyl ammonium; Z is selected from --O--,--COO--, --OOC--, --CONH--, and --NHCO--; R₁, R₂, R₃ are independently selected from hydrogen and methyl; R₄ is selected from methyl and ethyl; and R₅ is selected from hydrogen, methyl, ethyl, butyl, ethylhexyl, decyl, dodecyl, hydroxyethyl, hydroxypropyl, polyoxyethylene, and polyoxypropylene. Vinyl-functional cationic monomers suitably include, but are not limited to, [2-(acryloxy)ethyl] trimethyl ammonium chloride (ADAMQUAT); [2-(methacryloxy)ethyl) trimethyl ammonium chloride (MADQUAT); (3-acrylamidopropyl) trimethyl ammonium chloride; N,N-diallyldimethyl ammonium chloride; [2-(acryloxy)ethyl] dimethylbenzyl ammonium chloride; (2-(methacryloxy)ethyl] dimethylbenzyl ammonium chloride; [2-(acryloxy)ethyl] dimethyl ammonium chloride; [2-(methacryloxy)ethyl] dimethyl ammonium chloride. Precursor monomers, such as vinylpyridine, dimethylaminoethyl acrylate, and dimethylaminoethyl methacrylate, which can be polymerized and quaternized through post-polymerization reactions are also possible. Monomers or quaternization reagents which provide different counter-ions, such as bromide, iodide, or methyl sulfate are also useful. Other vinyl-functional cationic monomers which may be copolymerized with a hydrophobic vinyl monomer are also useful in the present disclosure.

Suitable hydrophobic monomers for use in the ion-sensitive cationic polymers include, but are not limited to, branched or linear C₁-C₁₈ alkyl vinyl ethers, vinyl esters, acrylamides, acrylates, and other monomers that can be copolymerized with the cationic monomer. As used herein the monomer methyl acrylate is considered to be a hydrophobic monomer. Methyl acrylate has a solubility of 6 g/100 ml in water at 20° C.

In one particularly suitable embodiment, the binder is the polymerization product of a cationic acrylate or methacrylate and one or more alkyl acrylates or methacrylates having the generic structure: 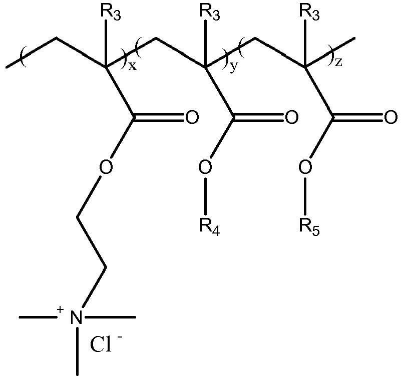 wherein x=1 to about 15 mole percent; y=about 60 to about 99 mole percent; and z=0 to about 30 mole percent; R₄ is selected from methyl and ethyl; R₅ is selected from hydrogen, methyl, ethyl, butyl, ethylhexyl, decyl, dodecyl, hydroxyethyl, hydroxypropyl, polyoxyethylene, and polyoxypropylene.

In a particularly suitable embodiment of the present disclosure, the ion triggerable polymer has the structure: 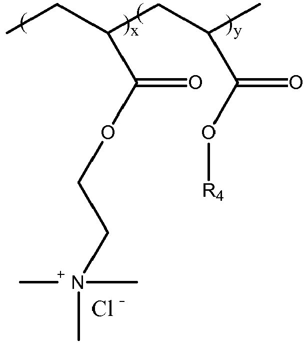 wherein x=1 to about 15 mole percent; y=about 85 to about 99 mole percent and R₄ is C₁-C₄ alkyl. In one particular embodiment, when R₄ is methyl, x=3 to about 6 mole percent; and y=about 94 to about 97 mole percent.

The ion triggerable cationic polymers may have an average molecular weight that varies depending on the ultimate use of the polymer. The ion triggerable cationic polymers have a weight average molecular weight ranging from about 10,000 to about 5,000,000 grams per mol. More specifically, the ion triggerable cationic polymers have a weight average molecular weight ranging from about 25,000 to about 2,000,000 grams per mole, or, more specifically still, from about 200,000 to about 1,000,000 grams per mole.

The ion triggerable cationic polymers may be prepared according to a variety of polymerization methods, a suitable method being a solution polymerization method. Suitable solvents for the polymerization method include, but are not limited to, lower alcohols, such as methanol, ethanol and propanol; a mixed solvent of water and one or more lower alcohols mentioned above; and a mixed solvent of water and one or more lower ketones, such as acetone or methyl ethyl ketone.

In the polymerization methods, any free radical polymerization initiator may be used. Selection of a particular initiator may depend on a number of factors including, but not limited to, the polymerization temperature, the solvent, and the monomers used. Suitable polymerization initiators for use in the present disclosure include, but are not limited to, 2,2'-azobisisobutyronitrile, 2,2'-azobis(2-methylbutyronitrile), 2,2'-azobis(2,4-dimethylvaleronitrile), 2,2'-azobis(2-amidinopropane)dihydrochloride, 2,2'-azobis(N,N'-dimethyleneisobutylamidine), potassium persulfate, ammonium persulfate, and aqueous hydrogen peroxide. The amount of polymerization initiator may suitably range from about 0.01 to 5 weight percent based on the total weight of monomer present.

The polymerization temperature may vary depending on the polymerization solvent, monomers, and initiator used, but in general, ranges from about 20° C to about 90° C. Polymerization time generally ranges from about 2 to about 8 hours.

The above-described ion triggerable cationic polymer formulations may be used as binder materials for flushable and/or non-flushable products. In order to be effective as a binder material in flushable products throughout the United States, the ion triggerable cationic polymer formulations remain stable and maintain their integrity while dry or in relatively high concentrations of a composition including at least one polyprotic acid having three or more functional groups and benzoic acid but become soluble in water containing up to about 200 ppm or more divalent ions, especially calcium and magnesium. Suitably, the ion triggerable cationic polymer formulations are insoluble in a solution containing from about 0.10 wt% to about 0.35 wt% of the polyprotic acid, including from about 0.20 wt% to about 0.35 wt%, and including about 0.20 wt% of the at least one polyprotic acid and from about 0.60 wt% to about 1.30 wt% of the benzoic acid, including from about 0.8 wt% to about 1.30 wt%, including 0.8 wt% to about 1.2 wt%, including from about 0.8 wt% to about 1.1 wt%, and including from about 0.8 wt% to about 1.0 wt%.

Based on a study conducted by the American Chemical Society, water hardness across the United States varies greatly, with CaCO₃ concentration ranging from near zero for soft water to about 500 ppm CaCO₃ (about 200 ppm Ca²⁺ ion) for very hard water. To ensure polymer formulation dispersibility across the country (and throughout the whole world), the ion triggerable cationic polymer formulations are suitably soluble in water containing up to 50 ppm Ca²⁺ and/or Mg²⁺ ions. More suitably, the ion triggerable cationic polymer formulations are soluble in water containing up to 100 ppm Ca²⁺ and/or Mg²⁺ ions. Even more suitably, the ion triggerable cationic polymer formulations are soluble in water containing up to 150 ppm Ca²⁺ and/or Mg²⁺ ions. Even more suitably, the ion triggerable cationic polymer formulations are soluble in water containing up to 200 ppm Ca²⁺ and/or Mg²⁺ ions.

### Co-binder Polymers

As stated above, the cationic polymer formulations are formed from a single triggerable cationic polymer or a combination of two or more different polymers, wherein at least one polymer is a triggerable polymer. The second polymer may be a co-binder polymer. A co-binder polymer is of a type and in an amount such that when combined with the triggerable cationic polymer, the co-binder polymer suitably is largely dispersed in the triggerable cationic polymer; i.e., the triggerable cationic polymer is suitably the continuous phase and the co-binder polymer is suitably the discontinuous phase. Suitably, the co-binder polymer can also meet several additional criteria. For example, the co-binder polymer can have a glass transition temperature; i.e., T_{g}, that is lower than the glass transition temperature of the ion triggerable cationic polymer. Furthermore or alternatively, the co-binder polymer can be insoluble in water, or can reduce the shear viscosity of the ion triggerable cationic polymer. The co-binder can be present at a level relative to the solids mass of the triggerable polymer of about 45% or less, specifically about 30% or less, more specifically about 20% or less, more specifically still about 15% or less, and most specifically about 10% or less, with exemplary ranges of from about 1% to about 45% or from about 25% to about 35%, as well as from about 1% to about 20% or from about 5% to about 25%. The amount of co-binder present should be low enough, for co-binders with the potential to form water insoluble bonds or films, that the co-binder remains a discontinuous phase unable to create enough crosslinked, or insoluble bonds, to jeopardize the dispersibility of a treated substrate.

Suitably, but not necessarily, the co-binder polymer when combined with the ion triggerable cationic polymer will reduce the shear viscosity of the ion triggerable cationic polymer to such an extent that the combination of the ion triggerable cationic polymer and the co-binder polymer is sprayable. By "sprayable" is meant that the polymer can be applied to a nonwoven fibrous substrate by spraying and the distribution of the polymer across the substrate and the penetration of the polymer into the substrate are such that the polymer formulation is uniformly applied to the substrate.

In some embodiments, the combination of the ion triggerable cationic polymer and the co-binder polymer can reduce the stiffness of the substrate to which it is applied compared to the substrate with just the ion triggerable cationic polymer.

The co-binder polymer can have an average molecular weight, which varies depending on the ultimate use of the polymer. Suitably, the co-binder polymer has a weight average molecular weight ranging from about 500,000 to about 200,000,000 grams per mol. More suitably, the co-binder polymer has a weight average molecular weight ranging from about 500,000 to about 100,000,000 grams per mol.

The co-binder polymer can be in the form of an emulsion latex. The surfactant system used in such a latex emulsion should be such that it does not substantially interfere with the dispersibility of the ion triggerable cationic polymer. Therefore, weakly anionic, nonionic, or cationic latexes may be useful for the present disclosure. In one embodiment, the ion triggerable cationic polymer formulations includes about 55 to about 95 weight percent ion triggerable cationic polymer and about 5 to about 45 weight percent poly(ethylene-vinyl acetate). More suitably, the ion triggerable cationic polymer formulations comprises about 75 weight percent ion triggerable cationic polymer and about 25 weight percent poly(ethylene-vinyl acetate). A particularly suitable non-crosslinking poly(ethylenevinyl acetate) is Dur-O-Set® RB available from National Starch and Chemical Co., Bridgewater, N.J.

When a latex co-binder, or any potentially crosslinkable co-binder, is used the latex should be prevented from forming substantial water-insoluble bonds that bind the fibrous substrate together and interfere with the dispersibility of the article. Thus, the latex can be free of crosslinking agents, such as N-methylol-acrylamide (NMA), or free of catalyst for the crosslinker, or both. Alternatively, an inhibitor can be added that interferes with the crosslinker or with the catalyst such that crosslinking is impaired even when the article is heated to normal crosslinking temperatures. Such inhibitors can include free radical scavengers, methyl hydroquinone, t-butylcatechol, pH control agents such as potassium hydroxide, and the like. For some latex crosslinkers, such as N-methylol-acrylamide (NMA), for example, elevated pH such as a pH of 8 or higher can interfere with crosslinking at normal crosslinking temperatures (e.g., about 130° C or higher). Also alternatively, an article comprising a latex co-binder can be maintained at temperatures below the temperature range at which crosslinking takes place, such that the presence of a crosslinker does not lead to crosslinking, or such that the degree of crosslinking remains sufficiently low that the dispersibility of the article is not jeopardized. Also alternatively, the amount of crosslinkable latex can be kept below a threshold level such that even with crosslinking, the article remains dispersible. For example, a small quantity of crosslinkable latex dispersed as discrete particles in an ion-sensitive binder can permit dispersibility even when fully crosslinked. For the later embodiment, the amount of latex can be below 20 weight percent, and, more specifically, below 15 weight percent relative to the ion-sensitive binder.

Latex compounds, whether crosslinkable or not, need not be the co-binder. SEM micrography of successful ion-sensitive binder films with useful non-crosslinking latex emulsions dispersed therein has shown that the latex co-binder particles can remain as discrete entities in the ion-sensitive binder, possibly serving in part as filler material. It is believed that other materials could serve a similar role, including a dispersed mineral or particulate filler in the triggerable binder, optionally comprising added surfactants/dispersants. For example, in one embodiment, freeflowing Ganzpearl PS-8F particles from Presperse, Inc. (Piscataway, N.J.), a styrene/divinylbenzene copolymer with about 0.4 micron particles, can be dispersed in a triggerable binder at a level of from about 2 to 10 weight percent to modify the mechanical, tactile, and optical properties of the triggerable binder. Other filler-like approaches may include microparticles, microspheres, or microbeads of metal, glass, carbon, mineral, quartz, and/or plastic, such as acrylic or phenolic, and hollow particles having inert gaseous atmospheres sealed within their interiors. Examples include EXPANCEL phenolic microspheres from Expancel of Sweden, which expand substantially when heated, or the acrylic microspheres known as PM 6545 available from PQ Corporation of Pennsylvania. Foaming agents, including CO₂ dissolved in the triggerable binder, could also provide helpful discontinuities as gas bubbles in the matrix of a triggerable binder, allowing the dispersed gas phase in the triggerable binder to serve as the co-binder. In general, any compatible material that is not miscible with the binder, especially one with adhesive or binding properties of its own, can be used as the co-binder, if it is not provided in a state that imparts substantial covalent bonds joining fibers in a way that interferes with the water-dispersibility of the product. However, those materials that also provide additional benefits, such as reduced spray viscosity, can be especially suitable. Adhesive co-binders, such as latex that do not contain crosslinkers or contain reduced amounts of crosslinkers, have been found to be especially helpful in providing good results over a wide range of processing conditions, including drying at elevated temperatures.

The co-binder polymer can comprise surface active compounds that improve the wettability of the substrate after application of the binder mixture. Wettability of a dry substrate that has been treated with a triggerable polymer formulation can be a problem in some embodiments, because the hydrophobic portions of the triggerable polymer formulation can become selectively oriented toward the air phase during drying, creating a hydrophobic surface that can be difficult to wet when the wetting composition is later applied unless surfactants are added to the wetting composition. Surfactants, or other surface active ingredients, in co-binder polymers can improve the wettability of the dried substrate that has been treated with a triggerable polymer formulation. Surfactants in the co-binder polymer should not significantly interfere with the triggerable polymer formulation. Thus, the binder should maintain good integrity and tactile properties in the pre-moistened wipes with the surfactant present.

In one embodiment, an effective co-binder polymer replaces a portion of the ion triggerable cationic polymer formulation and permits a given strength level to be achieved in a pre-moistened wipe with at least one of lower stiffness, better tactile properties (e.g., lubricity or smoothness), or reduced cost, relative to an otherwise identical pre-moistened wipe lacking the co-binder polymer and comprising the ion triggerable cationic polymer formulation at a level sufficient to achieve the given tensile strength.

Other co-binder polymers as known in the dispersible product art may be used without departing from the present disclosure. Such suitable co-binder polymers are disclosed, for example, in U.S. Patent No. 7,157,389 assigned to Kimberley-Clark.

### Binder Formulations and Fabrics Containing the Same

The ion triggerable cationic polymer formulations may be used as binders. The triggerable binder formulations may be applied to any fibrous substrate. The binders are particularly suitable for use in water-dispersible products. Suitable fibrous substrates include, but are not limited to, nonwoven and woven fabrics. In many embodiments, particularly personal care products, preferred substrates are nonwoven fabrics. As used herein, the term "nonwoven fabric" refers to a fabric that has a structure of individual fibers or filaments randomly arranged in a mat-like fashion (including papers). Nonwoven fabrics can be made from a variety of processes including, but not limited to, air-laid processes, wet-laid processes, hydroentangling processes, staple fiber carding and bonding, and solution spinning.

The triggerable binder composition may be applied to the fibrous substrate by any known process of application. Suitable processes for applying the binder material include, but are not limited to, printing, spraying, electrostatic spraying, coating, flooded nips, metered press rolls, impregnating or by any other technique. The amount of binder composition may be metered and distributed uniformly within the fibrous substrate or may be non-uniformly distributed within the fibrous substrate. The binder composition may be distributed throughout the entire fibrous substrate or it may be distributed within a multiplicity of small closely spaced areas. In most embodiments, uniform distribution of binder composition is desired.

For ease of application to the fibrous substrate, the triggerable binder may be dissolved in water, or in a non-aqueous solvent, such as methanol, ethanol, acetone, or the like, with water being the preferred solvent. The amount of binder dissolved in the solvent may vary depending on the polymer used and the fabric application. Suitably, the binder solution contains up to 50 percent by weight of binder composition solids. More suitably, the binder solution contains from about 10 to 30 percent by weight of binder composition solids, especially about 15 to about 25 percent by weight binder composition solids. Plasticizers, perfumes, coloring agents, antifoams, bactericides, preservative, surface active agents, thickening agents, fillers, opacifiers, tackifiers, detackifiers, and similar additives can be incorporated into the solution of binder components, if so desired.

Once the triggerable binder composition is applied to the substrate, the substrate is dried by any conventional means. Once dry, the coherent fibrous substrate exhibits improved tensile strength when compared to the tensile strength of the untreated wet-laid or dry-laid substrates, and yet has the ability to rapidly "fall apart", or disintegrate when placed in soft or hard water having a divalent ion concentration up to 200 ppm and agitated. For example, the dry tensile strength of the fibrous substrate may be increased by at least 25 percent as compared to the dry tensile strength of the untreated substrate not containing the binder. More particularly, the dry tensile strength of the fibrous substrate may be increase by at least 100 percent as compared to the dry tensile strength of the untreated substrate not containing the binder. Even more particularly, the dry tensile strength of the fibrous substrate may be increased by at least 500 percent as compared to the dry tensile strength of the untreated substrate not containing the binder.

Suitably, the improvement in tensile strength is effected where the amount of binder composition present, "add-on", in the resultant fibrous substrate represents only a small portion by weight of the entire substrate. The amount of "add-on" can vary for a particular application; however, the optimum amount of "add-on" results in a fibrous substrate which has integrity while in use and also quickly disperses when soaked in water. For example, the binder components typically are from about 5 to about 65 percent, by weight, of the total weight of the substrate. More particularly, the binder components may be from about 7 to about 35 percent, by weight, of the total weight of the substrate. Even more particularly, the binder components may be from about 10 to about 20 percent by weight of the total weight of the substrate.

The nonwoven fabrics have good in-use tensile strength, as well as, ion triggerability. Suitably, the nonwoven fabrics are abrasion resistant and retain significant tensile strength in aqueous solutions containing the specific amount and type of ions disclosed above. Because of this latter property, nonwoven fabrics are well suited for disposable products, such as sanitary napkins, diapers, adult incontinence products, and dry and premoistened wipes (wet wipes), which can be thrown in a flush toilet after use in any part of the world.

The fibers forming the fabrics above can be made from a variety of materials including natural fibers, synthetic fibers, and combinations thereof. The choice of fibers depends upon, for example, the intended end use of the finished fabric and fiber cost. For instance, suitable fibrous substrates may include, but are not limited to, natural fibers such as cotton, linen, jute, hemp, wool, wood pulp, etc. Similarly, regenerated cellulosic fibers, such as viscose rayon and cuprammonium rayon, modified cellulosic fibers, such as cellulose acetate, or synthetic fibers, such as those derived from polypropylenes, polyethylenes, polyolefins, polyesters, polyamides, polyacrylics, etc., alone or in combination with one another, may likewise be used. Blends of one or more of the above fibers may also be used, if so desired. Among wood pulp fibers, any known papermaking fibers may be used, including softwood and hardwood fibers. Fibers, for example, may be chemically pulped or mechanically pulped, bleached or unbleached, virgin or recycled, high yield or low yield, and the like. Mercerized, chemically stiffened or crosslinked fibers may also be used.

Synthetic cellulose fiber types include rayon in all its varieties and other fibers derived from viscose or chemically modified cellulose, including regenerated cellulose and solvent-spun cellulose, such as Lyocell. Chemically treated natural cellulosic fibers can be used, such as mercerized pulps, chemically stiffened or crosslinked fibers, or sulfonated fibers. Recycled fibers, as well as virgin fibers, can be used. Cellulose produced by microbes and other cellulosic derivatives can be used. As used herein, the term "cellulosic" is meant to include any material having cellulose as a major constituent, and, specifically, comprising at least 50 percent by weight cellulose or a cellulose derivative. Thus, the term includes cotton, typical wood pulps, non-woody cellulosic fibers, cellulose acetate, cellulose triacetate, rayon, thermomechanical wood pulp, chemical wood pulp, debonded chemical wood pulp, milkweed, or bacterial cellulose.

The triggerable binder may also be applied to other fibers or particles. Other fibers that may be treated with the triggerable binder include fiber such as those made fibers made from carboxymethyl cellulose, chitin, and chitosan. The triggerable binder may also be applied to particles, such as sodium polyacrylate super absorbent particles. Super absorbent particles are frequently incorporated on or into fibrous substrates used for personal care items, especially nonwoven fabrics.

Minimum length of the fibers typically depends on the method selected for forming the fibrous substrate. For example, where the fibrous substrate is formed by carding, the length of the fiber should usually be at least about 42 mm in order to insure uniformity.

Where the fibrous substrate is formed by air-laid or wet-laid processes, the fiber length may suitably be about 0.2 to 6 mm. Although fibers having a length of greater than 50 mm are suitable, it has been determined that when a substantial quantity of fibers having a length greater than 15 mm is placed in a flushable fabric, though the fibers will disperse and separate in water, their length tends to form "ropes" of fibers, which are undesirable when flushing in home toilets. Therefore, for these products, it is suitable that the fiber length be about 15 mm or less so that the fibers will not have a tendency to "rope" when they are flushed through a toilet. The fibers, particularly synthetic fibers, can also be crimped.

The fabrics may be formed from a single layer or multiple layers. In the case of multiple layers, the layers are generally positioned in a juxtaposed or surface-to-surface relationship and all or a portion of the layers may be bound to adjacent layers. Nonwoven webs may also be formed from a plurality of separate nonwoven webs wherein the separate nonwoven webs may be formed from single or multiple layers. In those instances where the nonwoven web includes multiple layers, the entire thickness of the nonwoven web may be subjected to a binder application or each individual layer may be separately subjected to a binder application and then combined with other layers in a juxtaposed relationship to form the finished nonwoven web.

In one embodiment, the fabric substrates may be incorporated into cleansing and body fluid absorbent products, such as sanitary napkins, diapers, adult incontinence products, surgical dressings, tissues, wet wipes, and the like. These products may include an absorbent core, comprising one or more layers of an absorbent fibrous material. The core may also comprise one or more layers of a fluid-pervious element, such as fibrous tissue, gauze, plastic netting, etc. These are generally useful as wrapping materials to hold the components of the core together. Additionally, the core may comprise a fluid-impervious element or barrier means to preclude the passage of fluid through the core and on the outer surfaces of the product. Suitably, the barrier means also is water-dispersible. A film of a polymer having substantially the same composition as the aforesaid water-dispersible binder is particularly well-suited for this purpose. The polymer compositions are useful for forming each of the above-mentioned product components including the layers of absorbent core, the fluid-pervious element, the wrapping materials, and the fluid-impervious element or barrier means.

In one embodiment of the present disclosure, the in-use tensile strength of a nonwoven fabric is enhanced by forming the nonwoven fabric with a binder material comprising the ion triggerable cationic polymer formulation and subsequently applying a wetting composition comprising at least one polyprotic acid having three or more functional groups and benzoic acid to the nonwoven fabric. The wetting composition may be applied to the nonwoven fabric by any method known to those of ordinary skill in the art including, but not limited to, applying a solid powder onto the fabric and spraying the wetting composition onto the fabric. The amount of polyprotic acid and benzoic acid may vary depending on a particular application. However, the amount of polyprotic acid applied to the fabric is typically from about 0.10 wt% to about 0.35 wt%, including from about 0.20 wt% to about 0.35 wt%, and including about 0.20 wt%. The amount of benzoic acid applied to the fabric is typically from about 0.60 wt% to about 1.30 wt% of the benzoic acid, including from about 0.8 wt% to about 1.30 wt%, including 0.8 wt% to about 1.2 wt%, including from about 0.8 wt% to about 1.1 wt%, and including from about 0.8 wt% to about 1.0 wt%.

Unlike other binder systems known in the art, the ion triggerable cationic polymer formulations can be activated as binders without the need for elevated temperature. While drying or water removal is useful in achieving a good distribution of the binder in a fibrous web, elevated temperature, per se, is not essential because the binder does not require crosslinking or other chemical reactions with high activation energy to serve as a binder. Rather, the interaction with a soluble insolubilizing compound, and specifically at least one polyprotic acid having at least three functional groups and benzoic acid, is sufficient to cause the binder to become insoluble; that is, activated by interaction between the cation of the polymer the polyprotic acid and benzoic acid. Thus, a drying step can be avoided, if desired, or replaced with low-temperature water removal operations such as room-temperature drying or freeze drying. Elevated temperature is generally helpful for drying, but the drying can be done at temperatures below what is normally needed to drive crosslinking reactions. Thus, the peak temperature to which the substrate is exposed or to which the substrate is brought can be below any of the following: 200° C, 180° C, 160° C, 140° C, 120° C, 110° C, 105° C, 100° C, 90° C, 75° C, and 60° C. While polymer systems, such as commercial latex emulsions, may also comprise crosslinkers suited for reaction at temperatures of 160° C or higher, maintaining a lower peak temperature can be beneficial in preventing development of excessive strength in the polymer that might otherwise hinder the water dispersibility of the pre-moistened wipe.

### Method of Making Wet Wipes

The pre-moistened wipes of the present disclosure, also referred to herein as wet wipes or flushable wipes, can be made in several ways. In one embodiment, the triggerable polymer composition is applied to a fibrous substrate as part of an aqueous solution or suspension, wherein subsequent drying is needed to remove the water and promote binding of the fibers. In particular, during drying, the binder migrates to the crossover points of the fibers and becomes activated as a binder in those regions, thus providing acceptable strength to the substrate. For example, the following steps can be applied:
1. Providing an absorbent substrate that is not highly bonded (e.g., an unbonded airlaid, a tissue web, a carded web, fluff pulp, etc.).
2. Applying a triggerable polymer composition to the substrate, typically in the form of a liquid, suspension, or foam.
3. Drying the substrate to promote bonding of the substrate. The substrate may be dried such that the peak substrate temperature does not exceed about 100° to 220° C.
4. Applying a wetting composition to the substrate.
5. Placing the wetted substrate in roll form or in a stack and packaging the product.

Application of the triggerable polymer composition to the substrate can be by means of spray; by foam application; by immersion in a bath; by curtain coating; by coating and metering with a wire-wound rod; by passage of the substrate through a flooded nip; by contact with a pre-metered wetted roll coated with the binder solution; by pressing the substrate against a deformable carrier containing the triggerable polymer composition such as a sponge or felt to effect transfer into the substrate; by printing such as gravure, inkjet, or flexographic printing; and any other means known in the art.

In the use of foams to apply a binder or co-binder polymer, the mixture is frothed, typically with a foaming agent, and spread uniformly on the substrate, after which vacuum is applied to pull the froth through the substrate. Any known foam application method can be used, including that of U.S. Pat. No. 4,018,647, "Process for the Impregnation of a Wet Fiber Web with a Heat Sensitized Foamed Latex Binder," issued Apr. 19, 1977 to Wietsma. Wietsma discloses a method wherein a foamed latex is heat-sensitized by the addition of a heat-sensitizer such as functional siloxane compounds including siloxane oxyalkylene block copolymers and organopolysiloxanes. Specific examples of applicable heat-sensitizers and their use thereof for the heat sensitization of latices are described in U.S. Pat. Nos. 3,255,140; 3,255,141; 3,483,240 and 3,484,394. The use of a heat-sensitizer is said to result in a product having a very soft and textile-like hand compared to prior methods of applying foamed latex binders.

The amount of heat-sensitizer to be added is dependent on, inter alia, the type of latex used, the desired coagulation temperature, the machine speed and the temperatures in the drying section of the machine, and will generally be in the range of about 0.05 to about 3% by weight, calculated as dry matter on the dry weight of the latex; but also larger or smaller amounts may be used. The heat sensitizer can be added in such an amount that the latex will coagulate far below the boiling point of water, for instance at a temperature in the range of about 35° C to about 95° C, or from about 35° C to 65° C.

Without wishing to be bound by theory, it is believed that a drying step after application of the triggerable binder solution and before application of the wetting composition enhances bonding of a fibrous substrate by driving the binder to fiber crossover points as moisture is driven off, thus promoting efficient use of the binder. However, in an alternative method, the drying step listed above is skipped, and the triggerable polymer composition is applied to the substrate followed by application of the wetting composition without significant intermediate drying. In one version of this method, the triggerable polymer composition selectively adheres to the fibers, permitting excess water to be removed in an optional pressing step without a significant loss of the binder from the substrate. In another version, no significant water removal occurs prior to application of the wetting composition. In yet another alternative method, the triggerable polymer composition and the wetting composition are applied simultaneously, optionally with subsequent addition of salt or other insolubilizing compounds to further render the binder insoluble.

### EXAMPLE 1

The objective of this Example was to determine alternatives to sodium chloride for use as a triggering agent in wetting compositions useful in wet wipes comprising an ion-sensitive binder composition.

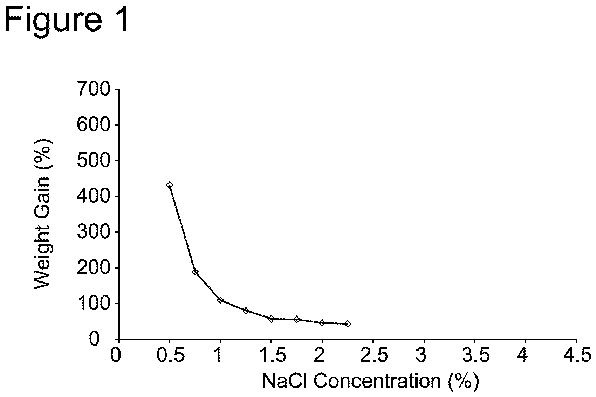

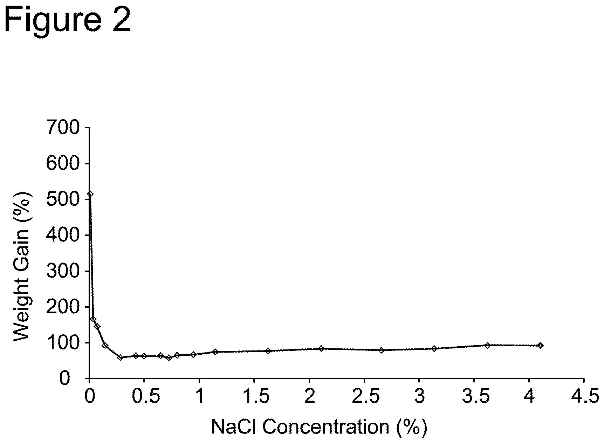

Aqueous solution of cationic polyacrylate that is the polymerization product of 96 mol% methyl acrylate and 4 mol% [2-(acryloyloxy)ethyl]trimethyl ammonium chloride was cast onto a Teflon® template to prepare air dried films. Samples were air dried and stored at ambient in a laboratory hood. Dry time before testing was greater than one week. Quantitative polymer film samples (approximately 1 inch by 1 inch) were placed in a series of nine 4-ounce glass jars containing 100 g of a solution of electrolyte (e.g., sodium chloride solution or sodium citrate solution) at concentrations varying from 0.5% to 2.25% for sodium chloride and from 0.01% to 4.5% for sodium citrate, respectively, in deionized water. Films were allowed to equilibrate in these solutions for 24 hours. The films were then removed from the solutions, slightly dabbed with a dry laboratory paper towel, and weighed (Percent weight gain = weight of water absorbed/original binder film weight * 100). The percent weight gain of the films was plotted versus the sodium chloride concentration (Figure 1) and the sodium citrate concentration (Figure 2) of solutions, respectively. The 2.0 wt% sodium chloride solution provided a polymer film weight gain in the flat region of the curve where it is insoluble and retains good mechanical properties. Between 0.5 wt% and 1.0 wt% of sodium chloride in solution, the weight gain of the polymer film increases, and the film is fully soluble at low levels of sodium chloride in solution. The insoluble films equilibrated in higher sodium chloride concentrations were secondarily placed in deionized water to test for dispersibility in ion-free water. The solubility curve concisely identifies sodium chloride concentration regions for insolubility and sodium chloride concentration regions of full solubility.

A lack of transition to water solubility at the lower salt concentration was observed for sodium citrate as is illustrated in Figure 2. Additionally, the polymer films did not readily dissolve in ion-free water during the secondary solubility characterization. The sodium citrate is hypothesized to bind to the polymer in the binding composition and insolubilize the polymer. Further, the films secondarily placed in water were not initially soluble, but over extended periods of time and with mechanical action ultimately swelled and dissolved. This may suggest that the binding of sodium citrate is somewhat reversible. Wipes prepared with 1.0 wt% and 2.0 wt% sodium citrate in water gave target in-use strength, but did not quickly lose their strength during the three-hour soak test, which could lead one to conclude that the binder composition is insoluble in sodium citrate, even at lower concentrations. The binder composition is known to be soluble in ion-free water, and therefore, the solubility characteristics of the binder compositions in low sodium citrate concentrations (0.1% to 0.5%) are of interest. Figure 2 illustrates the solubility curve of the polymer films in the low concentration range of sodium citrate. The solubility curve demonstrates that sodium citrate retains the binder composition in an insoluble state down to 0.2% in solution.

### EXAMPLE 2

In this Example, various wetting compositions were prepared and applied to fibrous airlaid wet wipe substrates including the cationic binder of Example 1 at an add-on amount of 235 wt%.

The various wetting compositions are shown in the table below.

| | Ingredients in wetting composition (wt.%) | | | | | | | | |
|---|---|---|---|---|---|---|---|---|---|
| Sample | | | | | | | Surfactant | | Other Ingredients |
| | NaCl | Sodium Citrate | Citric Acid | Sodium Benzoate | Benzoate acid | Malic Acid | Plantapon ® LGC (Cognis GmbH, Germany) | Arlasilk EFA (Croda, United Kingdom) | |
| Control | 2.0 | - | - | 0.45 | - | 0.11 | 0.64 | - | Proplene glycol (0.5) |
| 1 | - | 0.08 | 0.02 | - | - | - | - | 0.5 | |
| 2 | - | 0.16 | 0.04 | - | - | - | - | 0.5 | Polysorbate 20 (0.3) |
| 3 | - | 0.24 | 0.06 | - | - | - | - | 0.5 | |
| 4 | - | 0.32 | 0.08 | - | - | - | - | 0.5 | Fragrance (0.06) |
| 5 | - | 0.4 | 0.1 | - | - | - | - | 0.5 | Neolone 950 (Rohm and Hass, |
| 6 | - | - | - | 0.4 | 0.1 | - | - | 0.5 | |
| 7 | - | 0.08 | 0.02 | 0.4 | 0.1 | - | - | 0.5 | Philadephia, PA) (0.1) |
| 8 | - | 0.16 | 0.04 | 0.4 | 0.1 | - | - | 0.5 | |
| 9 | - | 0.24 | 0.06 | 0.4 | 0.1 | - | - | 0.5 | Water (balance, 98.44-95.84) |
| 10 | - | 0.32 | 0.08 | 0.4 | 0.1 | - | - | 0.5 | |
| 11 | - | - | - | 0.6 | 0.15 | - | - | 0.5 | |
| 12 | - | 0.08 | 0.02 | 0.6 | 0.15 | - | - | 0.5 | |
| 13 | - | 0.16 | 0.04 | 0.6 | 0.15 | - | - | 0.5 | |
| 14 | - | 0.24 | 0.06 | 0.6 | 0.15 | - | - | 0.5 | |
| 15 | - | 0.32 | 0.08 | 0.6 | 0.15 | - | - | 0.5 | |
| 16 | - | - | - | 0.8 | 0.2 | - | - | 0.5 | |
| 17 | - | 0.08 | 0.02 | 0.8 | 0.2 | - | - | 0.5 | |
| 18 | - | 0.16 | 0.04 | 0.8 | 0.2 | - | - | 0.5 | |
| 19 | - | 0.24 | 0.06 | 0.8 | 0.2 | - | - | 0.5 | |
| 20 | - | 0.32 | 0.08 | 0.8 | 0.2 | - | - | 0.5 | |

The wipes wetted with the compositions were then analyzed for strength, dispersibility and expressed pH. A standard laboratory tensile tester (Instron 5564 tensile tester with Bluehill2 software) was used to characterize in-use tensile strength properties. A 100-newton load cell with pneumatic grip was utilized, and a gauge length of 3 inches and a crosshead speed of 12 inches/minute were employed. The peak load values (g/in.) of sample replicates were recorded, averaged, and reported as wet tensile strength (i.e., in-use).

Dispersibility, noted in the table below as 1h soak strength, was assessed by transferring wet wipe samples into an excess (4.1L) of deionized water and allowing them to soak for approximately 1 hour. Tensile strength was then measured for 1h-soak strength. The results are shown in the table below.

| | Property of Moist Wipe | | | | |
|---|---|---|---|---|---|
| Sample | In-use (g/in) | 1 h soak strength (g/in) | Expressed pH | Ionic Strength | (benzoic acid + sodium benzoate) : (citic acid and sodium citrate) |
| Control | 509 | 47 | 5.09 | 0.393 | - |
| 1 | 60 | 49 | 5.2 | 0.014 | - |
| 2 | 70 | 63 | 5.07 | 0.025 | - |
| 3 | 140 | 115 | 5.04 | 0.037 | - |
| 4 | 230 | 143 | 5.04 | 0.049 | - |
| 5 | 290 | 219 | 5.04 | 0.051 | - |
| 6 | 69 | 35 | 5.24 | 0.037 | - |
| 7 | 150 | 41 | 5.18 | 0.051 | 5 |
| 8 | 250 | 58 | 5.17 | 0.065 | 2.5 |
| 9 | 334 | 109 | 5.14 | 0.078 | 1.67 |
| 10 | 373 | 141 | 5.13 | 0.092 | 1.25 |
| 11 | 208 | 25 | 5.27 | 0.056 | - |
| 12 | 295 | 39 | 5.22 | 0.070 | 7.5 |
| 13 | 364 | 64 | 5.2 | 0.083 | 3.75 |
| 14 | 398 | 110 | 5.23 | 0.097 | 2.5 |
| 15 | 415 | 131 | 5.21 | 0.111 | 1.875 |
| 16 | 318 | 32 | 5.27 | 0.075 | - |
| 17 | 380 | 34 | 5.27 | 0.088 | 10 |
| 18 | 403 | 50 | 5.26 | 0.102 | 5 |
| 19 | 403 | 57 | 5.24 | 0.116 | 3.33 |
| 20 | 416 | 115 | 5.23 | 0.129 | 2.5 |

As shown in the table, only the combination of citrate (i.e., polyprotic acids and their salt form) and benzoate (i.e., benzoic acid and its salt form) provided both wet strength and dispersibility.

### EXAMPLE 3

In this Example, various wetting compositions were prepared using monoprotic acids and their salt forms and applied to airlaid wet wipe substrates including the cationic binder of Example 1 at an add-on amount of 235 wt%.

Particularly, the wetting compositions similar to those of Example 2 were prepared using monoprotic acids other than benzoate and their salt forms. The salt combinations of the various wetting compositions are shown in the table below. Further, the wipes wetted with the compositions were then analyzed for strength, dispersibility and expressed pH as discussed in Example 2. The results are shown in the table below.

| | Property of Moist Wipe | | |
|---|---|---|---|
| Salt combination | In use (g/in) | 1 h soak strength (g/in) | Expressed pH |
| 0.2% sodium citrate, 0.05% citric acid/0.64% sodium salicylate, 0.16% salicylic acid | 648 | 605 | 4.76 |
| 0.2% sodium citrate, 0.05% citric acid/0.96% sodium salicylate, 0.24% salicylic acid | 613 | 613 | 4.75 |
| 0.2% sodium citrate, 0.05% citric acid/0.64% sodium lactate, 0.16% lactic acid | 88 | - | 4.59 |
| 0.2% sodium citrate, 0.05% citric acid/0.96% sodium lactate, 0.24% lactic acid | 63 | - | 4.53 |
| 0.2% sodium citrate, 0.05% citric acid/0.64% sodium glycolate, 0.16% glycolic acid | 99 | - | 4.43 |
| 0.2% sodium citrate, 0.05% citric acid/0.96% sodium glycolate, 0.24% glycolic acid | 81 | - | 4.38 |

As shown in the table, a combination of polyprotic acids and their salt forms with other monoprotic acids such as salicylic acid, lactic acid, glycolic acid and their salt forms could not provide both wet strength and dispersability.

### EXAMPLE 4

In this Example, wetting compositions were prepared using various surfactants and applied to airlaid wet wipe substrates including the cationic binder of Example 1 at an add-on amount of 235 wt%.

The various wetting compositions are shown in the tables below. Further, the wipes wetted with the compositions were then analyzed for strength, dispersibility and expressed pH as described in Example 2. The results are also shown in the tables below.

| | Ingredients in wetting composition (wt.%) | | | | | | | | Property of Moist Wipe | |
|---|---|---|---|---|---|---|---|---|---|---|
| Sample | Surfactant | NaCl | Sodium citrate | Citric acid | Sodium benzoate | Benzoic acid | Malic acid | Other ingredients | In-use (g/in) | 1h soak (g/in) |
| Control | Plantapon LGC (Cognis GmbH, Germany) (0.64) | 2.0 | - | - | 0.45 | - | 0.11 | Propylene glycol (0.5) Polysorbate 20 (0.3) Cocoon | 438 | 81 |
| Citrate/benzoate | Plantapon LGC (Cognis GmbH, Germany) (0.5) | - | 0.2 | 0.05 | 0.8 | 0.2 | - | Fragrance (0.06) Neolone 950 (Rohm and Haas, Philadephia, PA) (0.1) | 353 | 200 |
| | Mackanate DC-30 (Rhodia Novecare, France) (0.5) | | | | | | | | 372 | 288 |
| | Mackanate DOS-70PG (Rhodia Novecare, France) (0.5) | | | | | | | | 421 | 372 |

Amphoteric (Zwitterionic) or Nonionic Surfactants

| | Ingredients in wetting composition (wt.%) | | | | | | | | | Property of Moist Wipe | |
|---|---|---|---|---|---|---|---|---|---|---|---|
| Sample | Surfactant | | NaCl | Sodium citrate | Citric acid | Sodium benzoate | Benzoic acid | Malic acid | Other ingredients | In-use (g/in) | 1h soak (g/in) |
| Control | Plantapon LGC (Cognis Gmbh, Germany) (0.64) | | 2.0 | - | - | 0.45 | - | 0.11 | Propylene glycol (0.5) Polysorbate 20 (0.3) Cocoon Fragrance (0.06) Neolone 950 (Rohm and Haas, Philadephia , PA) (0.1) | 530 | 84 |
| Citrate/ Benzoate | Amphoteric | Arlasilk PTM (Croda, United Kingdom) (0.5) | - | 0.2 | 0.05 | 0.8 | 0.2 | - | | 498 | 77 |
| | | | | | | | | | | 457 | 71 |
| | Nonionic | Plantacare 1200UP (BASF, Germany) (0.5) | | | | | | | | 500 | 76 |
| | | Glycerox HE (Croda, United Kingdom) (0.5) | | | | | | | | 458 | |

As shown in the above tables, anionic surfactants in combination with citrate and benzoate and salts thereof did not disperse well. However, combinations of amphoteric, nonionic and cationic surfactants with citrate and benzoate and salts thereof did disperse.

### EXAMPLE 5

In this Example, various wetting compositions including emulsions were prepared and applied to fibrous wet wipe substrates. The compositions were tested for emulsion stability and the substrates were evaluated for tensile strength and dispersibility.

To determine emulsion stability in the wetting compositions, 5 wt% of various emulsion solutions were prepared based on vendor instructions and mixed with either 2 wt% sodium chloride or a mixture of: 0.2 wt% sodium citrate, 0.05 wt% citric acid, 0.8 wt% sodium benzoate, and 0.2 wt% benzoic acid. Other ingredients included: 0.5% propylene glycol, 0.3% polysorbate 20, 0.06% cocoon fragrance, and 0.1% Neolone 950 (Rohm and Haas, Philadephia, PA). The emulsions were allowed to dissolve completely. After complete dissolution, all wetting compositions were placed in an oven at 50°C for 2 weeks. After 2 weeks, stability, in terms of phase separation of water and oil, were evaluated. The results are shown in the table below.

| Emulsion (5% conc.) | Stability w/ 2 wt% NaCl | Stability w/ Citrate/Benzoate combination |
|---|---|---|
| TEGO WIPE FLEX (Evonik Industries, Germany) | X | X |
| TEGO WIPE DE (Evonik Industries, Germany) | X | X |
| PhoenoMulse CE-1 (Phoenix Chemical, Inc., Somerville, NJ) | X | X |
| SMEC Natural (Arch Chemicals, Switzerland) | X | X |
| Plantasil Micro (BASF, Germany) | X | ○ |
| Incroquat Behenyl TMS-50 (Croda, United Kingdom) | X | ○ |
| SMEC Concentrate (Arch Chemicals, Switzerland) | ○ | ○ |
| Emulgade CPE (BASF, Germany) | ○ | ○ |

| | | |
|---|---|---|
| X = phase separation (unstable emulsion); O = no phase separation (stable emulsion) | | |

A standard lab protocol was used for preparing and conditioning the fibrous wet wipe samples. Specifically, a die was used to obtain 5.5 inch by 7.5 inch wipes from a quantity of standard basesheet as described above. A standard hand-pump spray bottle was used to apply 235% by weight average add-on of wetting composition including Incroquat Behenyl TMS-50, at various concentrations, by spraying each side of the individual sheets with five to seven pumps of spray. A set of wetted wipes was stacked and placed into a sealable plastic baggie for conditioning. All samples and baggies were placed under 0.05 psi load for a condition of two days prior to characterization.

A standard laboratory tensile tester (Instron 5564 tensile tester with Bluehill2 software) was used to characterize in-use tensile strength properties. A 100-newton load cell with pneumatic grip was utilized, and a gauge length of 3 inches and a crosshead speed of 12 inches/minute were employed. The peak load values (g/in.) of sample replicates were recorded, averaged, and reported as wet tensile strength (i.e., in-use).

Dispersibility, noted in the table below as 1h soak strength, was assessed by transferring wet wipe samples into an excess (4.1L) of deionized water and allowing them to soak for approximately 1 hour. Tensile strength was then measured for 1h-soak strength. Results of the tensile strength and dispersibility are shown in the table below.

| Sample | Emulsion Conc. | In-use ((g/in) | 1h soak (g/in ) | Expressed pH |
|---|---|---|---|---|
| Control | | 553 | 49 | 5.14 |
| TMS-50 Emulsion Solution | 0.0 | 551 | 91 | 5.39 |
| | 0.5 | 453 | 57 | 5.31 |
| | 1.0 | 470 | 75 | 5.37 |
| | 1.5 | 462 | 48 | 5.36 |

As shown in the tables above, by using a combination of citrate and benzoate in a wetting composition as taught in the present disclosure, the wetting composition can include emulsions that are normally not compatible with 2 wt% sodium chloride solutions. Further, these wetting compositions provide improved wet strength and dispersibility.

## Claims

1. A wet wipe comprising:
a fibrous material having a strength of at least 300 g/in and a dispersibility of no more than 100 g/in;
an ion-triggerable cationic binder composition; and
a wetting composition comprising:
at least one polyprotic acid having three or more functional groups and benzoic acid, wherein the ratio of the benzoic acid to the at least one polyprotic acid is greater than 2.5.

2. The wet wipe of claim 1 wherein the polyprotic acid is sodium citrate.

3. The wet wipe of claim 1 wherein the ratio of the benzoic acid to the at least one polyprotic acid is from about 3.0 to about 10.

4. The wet wipe of claim 1 wherein the ratio of the benzoic acid to the at least one polyprotic acid is from about 4 to about 4.5.

5. The wet wipe of claim 1 wherein the benzoic acid is present in an amount of from about 0.80 wt% to about 1.0 wt% by total weight of the wetting composition.

6. The wet wipe of claim 1 wherein the at least one polyprotic acid is present in an amount of about 0.20 wt% by total weight of the wetting composition.

7. The wet wipe of claim 1 wherein the pH of the wetting composition after being expressed from the wet wipe is from about 4.5 to about 6.0.

8. The wet wipe of claim 1 wherein the wetting composition comprises sodium citrate, citric acid, sodium benzoate, and benzoic acid.

9. The wet wipe of claim 1 wherein the wet wipe additionally comprises at least one component selected from the group consisting of cationic surfactants, nonionic surfactants, amphoteric surfactants, zwitterionic surfactants, and combinations thereof.

10. A wet wipe comprising:
a fibrous material having a strength of at least 300 g/in and a dispersibility of no more than 100 g/in;
an ion-triggerable cationic binder composition; and
a wetting composition comprising:
at least one polyprotic acid having three or more functional groups and benzoic acid, wherein the pH of the wetting composition after expression from the wet wipe is from about 4.5 to about 6.0.

11. A wet wipe comprising:
a fibrous material having a strength of at least 300 g/in and a dispersibility of no more than 100 g/in;
an ion-triggerable cationic binder composition; and
a wetting composition comprising:
at least one polyprotic acid having three or more functional groups, benzoic acid, behentrimonium methosulfate, cetearyl alcohol, and butylene glycol, wherein the ratio of the benzoic acid to the at least one polyprotic acid is greater than 2.5.

12. The wet wipe of claim 1, claim 10 or claim 11 wherein the at least one polyprotic acid having three or more functional groups is selected from the group consisting of sodium citrate, EDTA, and salts thereof.

13. The wet wipe of claim 1, claim 10 or claim 11 wherein the at least one polyprotic acid is present in an amount of from about 0.15 wt% to about 0.35 wt% by total weight of the wetting composition.

14. The wet wipe of claim 1, claim 10 or claim llwherein the benzoic acid is present in an amount of from about 0.60 wt% to about 1.30 wt% by total weight of the wetting composition.

## Patentansprüche

1. Feuchttuch, umfassend:
ein Fasermaterial mit einer Festigkeit von wenigstens 118 g/cm (300 g/in) und einer Dispergierbarkeit von nicht mehr als 39,4 g/cm (100 g/in);
eine ionenaktiverbare kationische Bindemittelzusammensetzung und
eine Benetzungszusammensetzung, umfassend:
wenigstens eine mehrbasige Säure mit drei oder mehr funktionellen Gruppen und Benzoesäure, wobei das Verhältnis von Benzoesäure zur wenigstens einen mehrbasigen Säure größer als 2,5 ist.

2. Feuchttuch nach Anspruch 1, wobei die mehrbasige Säure Natriumcitrat ist.

3. Feuchttuch nach Anspruch 1, wobei das Verhältnis von Benzoesäure zur wenigstens einen mehrbasigen Säure etwa 3,0 bis etwa 10 beträgt.

4. Feuchttuch nach Anspruch 1, wobei das Verhältnis von Benzoesäure zur wenigstens einen mehrbasigen Säure etwa 4 bis etwa 4,5 beträgt.

5. Feuchttuch nach Anspruch 1, wobei die Benzoesäure in einer Menge von etwa 0,80 Gew.-% bis etwa 1,0 Gew.-%, bezogen auf das Gesamtgewicht der Benetzungszusammensetzung, vorhanden ist.

6. Feuchttuch nach Anspruch 1, wobei die wenigstens eine mehrbasige Säure in einer Menge von etwa 0,20 Gew.-%, bezogen auf das Gesamtgewicht der Benetzungszusammensetzung, vorhanden ist.

7. Feuchttuch nach Anspruch 1, wobei der pH-Wert der Benetzungszusammensetzung nach ihrem Auspressen aus dem Feuchttuch etwa 4,5 bis etwa 6,0 beträgt.

8. Feuchttuch nach Anspruch 1, wobei die Benetzungszusammensetzung Natriumcitrat, Citronensäure, Natriumbenzoat und Benzoesäure umfasst.

9. Feuchttuch nach Anspruch 1, wobei das Feuchttuch darüber hinaus wenigstens eine Komponente umfasst, die ausgewählt ist aus der Gruppe bestehend aus kationischen Tensiden, nichtionischen Tensiden, amphoteren Tensiden, zwitterionischen Tensiden und Kombinationen davon.

10. Feuchttuch, umfassend:
ein Fasermaterial mit einer Festigkeit von wenigstens 118 g/cm (300 g/in) und einer Dispergierbarkeit von nicht mehr als 39,4 g/cm (100 g/in);
eine ionenaktiverbare kationische Bindemittelzusammensetzung und
eine Benetzungszusammensetzung, umfassend:
wenigstens eine mehrbasige Säure mit drei oder mehr funktionellen Gruppen und Benzoesäure, wobei der pH-Wert der Benetzungszusammensetzung nach dem Auspressen aus dem Feuchttuch etwa 4,5 bis etwa 6,0 beträgt.

11. Feuchttuch, umfassend:
ein Fasermaterial mit einer Festigkeit von wenigstens 118 g/cm (300 g/in) und einer Dispergierbarkeit von nicht mehr als 39,4 g/cm (100 g/in);
eine ionenaktiverbare kationische Bindemittelzusammensetzung und
eine Benetzungszusammensetzung, umfassend:
wenigstens eine mehrbasige Säure mit drei oder mehr funktionellen Gruppen, Benzoesäure, Behentrimoniummethosulfat, Cetylstearylalkohol und Butylenglycol, wobei das Verhältnis von Benzoesäure zur wenigstens einen mehrbasigen Säure größer als 2,5 ist.

12. Feuchttuch nach Anspruch 1, Anspruch 10 oder Anspruch 11, wobei die wenigstens eine mehrbasige Säure mit drei oder mehr funktionellen Gruppen ausgewählt ist aus der Gruppe bestehend aus Natriumcitrat, EDTA und Salzen davon.

13. Feuchttuch nach Anspruch 1, Anspruch 10 oder Anspruch 11, wobei die wenigstens eine mehrbasige Säure in einer Menge von etwa 0,15 Gew.-% bis etwa 0,35 Gew.-%, bezogen auf das Gesamtgewicht der Benetzungszusammensetzung, vorhanden ist.

14. Feuchttuch nach Anspruch 1, Anspruch 10 oder Anspruch 11, wobei die Benzoesäure in einer Menge von etwa 0,60 Gew.-% bis etwa 1,30 Gew.-%, bezogen auf das Gesamtgewicht der Benetzungszusammensetzung, vorhanden ist.

## Revendications

1. Lingette humide comprenant :
un matériau fibreux ayant une résistance d'au moins 300 g/pouce et une dispersibilité non supérieure à 100 g/pouce ;
une composition de liant cationique à déclenchement ionique ; et
une composition mouillante comprenant :
au moins un acide polyprotique ayant au moins trois groupes fonctionnels et de l'acide benzoïque, le rapport entre l'acide benzoïque et l'au moins un acide polyprotique étant supérieur à 2,5.

2. Lingette humide de la revendication 1, dans laquelle l'acide polyprotique est le citrate de sodium.

3. Lingette humide de la revendication 1, dans laquelle le rapport entre l'acide benzoïque et l'au moins acide polyprotique est d'environ 3,0 à environ 10.

4. Lingette humide de la revendication 1, dans laquelle le rapport entre l'acide benzoïque et l'au moins un acide polyprotique est d'environ 4 à environ 4,5.

5. Lingette humide de la revendication 1, dans laquelle l'acide benzoïque est présent dans une quantité d'environ 0,80 % en poids à environ 1,0 % en poids par rapport au poids total de la composition mouillante.

6. Lingette humide de la revendication 1, dans laquelle l'au moins un acide polyprotique est présent dans une quantité d'environ 0,20 % en poids par rapport au poids total de la composition mouillante.

7. Lingette humide de la revendication 1, dans laquelle le pH de la composition mouillante après avoir été extraite de la lingette humide est d'environ 4,5 à environ 6,0.

8. Lingette humide de la revendication 1, dans laquelle la composition mouillante comprend du citrate de sodium, de l'acide citrique, du benzoate de sodium et de l'acide benzoïque.

9. Lingette humide de la revendication 1, la lingette humide comprenant également au moins un composant choisi dans le groupe constitué par les agents tensioactifs cationiques, les agents tensioactifs non ioniques, les agents tensioactifs amphotères, les agents tensioactifs zwittérioniques et leurs combinaisons.

10. Lingette humide comprenant :
un matériau fibreux ayant une résistance d'au moins 300 g/pouce et une dispersibilité non supérieure à 100 g/pouce ;
une composition de liant cationique à déclenchement ionique ; et
une composition mouillante comprenant :
au moins un acide polyprotique ayant au moins trois groupes fonctionnels et de l'acide benzoïque, le pH de la composition mouillante après extraction depuis la lingette étant d'environ 4,5 à environ 6,0.

11. Lingette humide comprenant :
un matériau fibreux ayant une résistance d'au moins 300 g/pouce et une dispersibilité non supérieure à 100 g/pouce ;
une composition de liant cationique à déclenchement ionique ; et
une composition mouillante comprenant :
au moins un acide polyprotique ayant au moins trois groupes fonctionnels, de l'acide benzoïque, du méthosulfate de béhentrimonium, de l'alcool cétéarylique et du butylène glycol, le rapport entre l'acide benzoïque et l'au moins un acide polyprotique étant supérieur à 2,5.

12. Lingette humide de la revendication 1, de la revendication 10 ou de la revendication 11, dans laquelle l'au moins un acide polyprotique ayant au moins trois groupes fonctionnels est choisi dans le groupe constitué par le citrate de sodium, l'EDTA et leurs sels.

13. Lingette humide de la revendication 1, de la revendication 10 ou de la revendication 11, dans laquelle l'au moins un acide polyprotique est présent dans une quantité d'environ 0,15 % en poids à environ 0,35 % en poids par rapport au poids total de la composition mouillante.

14. Lingette humide de la revendication 1, de la revendication 10 ou de la revendication 11, dans laquelle l'acide benzoïque est présent dans une quantité d'environ 0,60 % en poids à environ 1,30 % en poids par rapport au poids total de la composition mouillante.
